# EUROPEAN PATENT SPECIFICATION

(11) **EP 0 819 017 B1**
(45) Date of publication and mention of the grant of the patent: **17.07.2002**
(21) Application number: 95916828.7
(22) Date of filing: 05.04.1995
(51) Int. Cl.: A61N 5/06

(54) **HEART BEAT SYNCHRONIZED INFRARED RADIATION EMISSION DEVICE**
MIT DER HERZFREQUENZ SYNCHRONISIERTE INFRAROTBESTRAHLUNGSVORRICHTUNG
DISPOSITIF EMETTEUR DE RAYONNEMENT INFRAROUGE SYNCHRONISE AVEC LE BATTEMENT CARDIAQUE

(43) Date of publication of application: 21.01.1998
(73) Proprietor: Digonzelli, Sergio, 23014 Delebio (IT); Bovo, Silvano, 23037 Tirano (IT)
(72) Inventor: BOVO, Silvano, I-23037 Tirano (IT)
(74) Representative: Cioni, Carlo
(86) International application number: IT9500046
(87) International publication number: WO9631252

(56) References cited:
- DE-U- 9 103 110
- MEDICAL & BIOLOGICAL ENGINEERING & COMPUTING, no. 6, 29 November 1991 STEVENAGE, pages 45-48, XP 000276069 MEFFERT 'Effects of a multiple mild infra-red-a'

## Description

### Technical field

The present invention relates to devices used in the radiation therapies and more particularly it concerns a device for the heart rate synchronized infrared radiation emission.

### Background art

It is well known that, besides many kind of drugs, infrared emission lamps are commonly used for the care of various pathologies. At the present time constant intensity radiation emission lamps or periodical intensity lowering radiation emission lamps are used. However, the therapeutic results with the above operating procedures are found to be generally unsatisfactory.

GB-A-1 320 030 discloses a device for the infrared radiation therapy including a series of lamps switched on and off in succession in order to create a wave of infrared emission such switching on and off in succession can be controlled by the patient pulse. DE-U-9 103 110.9 discloses an infrated radiation therapy device the light sources of which may be triggered by a bio-feedback system controlled by a pulse sensor.

The present invention aims to provide an infrared emission device enabling to obtain much better results.

### Disclosure of the invention

The object of the present invention is achieved with an infrared emission device equipped with an electronic circuit, controlling the infrared emission periodical cut-off and synchronizing such periodical cut-off with the heart rate.

Besides the care of various pathologies, the device according to the present invention can be successfully used for trichologic and aesthetic treatments.

The device according to the present invention comprises one or more conventional infrared lamps connected with an electronic circuit controlling the periodical cut-off of the above infrared lamp and synchronizing such cut-off with the heart rate. It has been found that the infrared lamp periodical cut-off synchronized with the heart rate provides remarkable advantages in terms of therapeutic, trichological and aesthetic efficiency over the use of conventional devices operating under constant or periodically attenuated infrared emission intensity or periodical complete infrared emission estinguishing unsynchronized with the heart rate.

The device of the invention operates at a frequency which is synchronized with the therapy subjected patient heart rate. For this purpose the device comprises further a patient heart rate detector.

The heart rate detectors are well known in the art and they will not be described in the present application.

The heart rate detector acts on the electronic circuit determining the periodical cut-off of the infrared radiation emission. As abovesaid the infrared lamps used in the device according to the invention are well known in the art. Preferably one or more 100 - 200 watt infrared lamps can be used even though lamps of different power are also satisfactory.

The device of the invention may also comprise a timer to define in advance the duration of the treatment.

Some examples of the use of the device according to the present invention are reported hereinafter.

### Example 1

Sex of the patient: male
Age of the patient: 72
Pathology: cervical arthrosis, cervical cartilage degenerative process.
Symptoms: sharp neck-aches, particularly during the usual neck movements
Therapy: one application of 20 minute/day for ten days. Each application was divided into ten minute diastole and ten minute systole synchronization.
Results: After three application pain was 30 - 50% attenuated and it disappeared at the end of the therapy. After six months from the therapy completion still no neckache was detected.

### Example 2

Sex of the patient: female
Age of the patient: 57
Pathology: Raynaud's disease, hand artery paroxysmal vasoconstriction resulting in a touch sensibility failure.
Therapy: one application of 15 minutes/day for 20 days, diastole synchronization.
Results: The first improvements were found after five applications. A progressive increase of the touch sensibility was revealed starting from the tenth application. Skin natural colour recovery after the thirteenth application. Complete recovery after seventeen applications. No disease symptoms have been revealed after six months from the therapy completion.

### Example 3.

Sex of the patient: Female
Age of the patient: 26
Pathology: Asthenia and lack of strength, debility.
Therapy:15 diastole/systole synchronised alternate applications of ten minutes each made in correspondence of the solar plexus, kidneys, liver and nape.
Results: Some improvements have been found after three applications. Progressive increase of strength after ten applications. The results remained stable after six months from the completion of therapy.

### Example 4

Permanent wave
Age of the client: 43
Operation modalities: 10 minutes of application with diastole synchronization. Reduction to 50% of the commonly used products and time.
Results: Fairly improved permanent wave with more beautiful, soft, and bright hairs.

### Example 5

Hair dying
Age of the client: 50
Operation modalities: 10 minutes of application with diastole synchronization. Reduction to 50% of the commonly used products and time.
Results: more brilliant hair colour, more bright, thick, strong and vivid hairs

### Example 6

Face cleaning
Age of the client: 37
Operation modalities: 10 minutes of application with alternate diastole/systole synchronization in presence of a suitable protection of the eyes.
Results: extremely high skin brightness and shining.

## Claims

1. A device for the infrared radiation emission comprising one or more infrared lamps and an electronic circuit periodically switching off completely said infrared lamp, **characterised in that** the electronic circuit synchronises the periodical switching off with the heart beat of the patient submitted to infrared emission therapy.

2. A device according to claim 1 in which each switching off of the infrared lamp occurs at the beginning of the systolic phase of the patient submitted to the infrared emission therapy.

3. A device according to claim 1 in which each switching off of the infrared lamps occurs at the beginning of the diastolic phase of the patient submitted to the infrared emission therapy.

4. A device according to claims 1 to 3, **characterised in that** it comprises a timer to determine the duration of infrared radiation treatment.

## Patentansprüche

1. Vorrichtung zur Abgabe von Infrarotstrahlung, die eine oder mehrere Infrarotlampen und einen elektrischen Schaltkreis umfaßt, der die Infrarotlampe in periodischen Abständen vollständig ausschaltet, **dadurch gekennzeichnet, dass** der elektronische Schaltkreis das periodische Ausschalten mit einem Herzschlag eines Patienten, der sich einer Infrarotstrahlentherapie unterzieht, synchronisiert.

2. Vorrichtung nach Anspruch 1, bei der jedes Ausschalten der Infrarotlampe zu Beginn einer systolischen Phase des Patienten stattfindet, der sich der Infrarotstrahlentherapie unterzieht.

3. Vorrichtung nach Anspruch 1, bei der jedes Ausschalten der Infrarotlampe zu Beginn einer diastolischen Phase des Patienten beginnt, der sich der Inftrarotstrahlentherapie unterzieht.

4. Vorrichtung nach einem der Ansprüche 1 bis 3, **dadurch gekennzeichnet, dass** sie einen Zeitgeber zum bestimmen der Dauer einer Infrarotstrahlenbehandlung umfasst.

## Revendications

1. Dispositif pour l'émission d'un rayonnement infrarouge comprenant une ou plusieurs lampes à infrarouges et un circuit électronique éteignant complètement périodiquement cette lampe à infrarouges, **caractérisé en ce que** le circuit électronique synchronise la coupure périodique de la lampe avec le battement du coeur du patient soumis à la thérapie basée sur l'émission dans l'infrarouge.

2. Dispositif selon la revendication 1, dans lequel chaque coupure de la lampe à infrarouges se produit au début de la phase systolique du patient soumis à la thérapie basée sur l'émission dans l'infrarouge.

3. Dispositif selon la revendication 1, dans lequel chaque coupure des lampes à infrarouges se produit au début de la phase diastolique du patient soumis à la thérapie basée sur l'émission dans l'infrarouge.

4. Dispositif selon les revendications 1 à 3, **caractérisé en ce qu'**il comporte un minuteur pour déterminer la durée du traitement au rayonnement infrarouge.
